Europäisches Patentamt

European Patent Office

Office européen des brevets

(19)

(11) **EP 1 579 857 A1**

(12) **EUROPEAN PATENT APPLICATION**

(43) Date of publication:
   **28.09.2005 Bulletin 2005/39**

(51) Int Cl.⁷: **A61K 31/138**, A61K 9/08,
   A61K 47/00, A61P 35/00

(21) Application number: **04290762.6**

(22) Date of filing: **22.03.2004**

(84) Designated Contracting States:
   **AT BE BG CH CY CZ DE DK EE ES FI FR GB GR
   HU IE IT LI LU MC NL PL PT RO SE SI SK TR**
   Designated Extension States:
   **AL LT LV MK**

(71) Applicant: **LABORATOIRES BESINS
   INTERNATIONAL
   75003 Paris (FR)**

(72) Inventors:
   • **Hilt, Dana C.
   Maryland 21042 (US)**

   • **Masini-Eteve, Valerie
   91370 Verrieres Le Buisson (FR)**
   • **Fedynec, Richard
   78120 Clairefontaine (FR)**
   • **Taravella, Brigitte
   75013 Paris (FR)**

(74) Representative: **Nargolwalla, Cyra
   Cabinet Plasseraud
   65/67 rue de la Victoire
   75440 Paris Cedex 09 (FR)**

(54) **Chemically stable compositions of 4-hydroxy tamoxifen**

(57)   A class of chemically stable 4-hydroxy tamoxifen compositions is characterized by roughly equal amounts of the *Z*-4-hydroxy tamoxifen and *E*-4-hydroxy tamoxifen isomers, such that the potency of the compositions is not affected adversely by the isomerization that occurs between these two forms. The inventive compositions are useful for preventing or for treating medical conditions.

EP 1 579 857 A1

**Description**

**Background of the Invention**

The present invention relates to chemically stable isomeric compositions of 4-hydroxy tamoxifen (4-OHT), an active metabolite of the drug tamoxifen.

Tamoxifen acts on estrogen receptors throughout the body and, as both an agonist and antagonist, provokes a wide range of systemic effects. It is widely prescribed for breast cancer because it blocks the effects of estrogen in breast tissue, thereby slowing or stopping the growth of cancer cells that are already present and preventing the development of new cancers. Because of its wide ranging effects, tamoxifen causes significant side effects, which increase the risk of endometrial cancer, endometrial hyperplasia and polyps, deep vein thrombosis and pulmonary embolism, changes in liver enzyme levels, and ocular toxicities, including cataracts. Additionally, patients treated with tamoxifen report having hot flashes, vaginal discharge, depression, amenorrhea, and nausea.

Due to tamoxifen's drawbacks, some cancer researchers have proposed substituting 4-hydroxy tamoxifen as a treatment for breast cancer. 4-Hydroxy tamoxifen acts as a selective estrogen receptor modulator (SERM) that exhibits tissue-specificity for estrogen receptive tissues. In breast tissue, it functions as an estrogen antagonist. Studies have shown that 4-hydroxy tamoxifen can regulate the transcriptional activity of estrogen-related receptors, which may contribute to its tissue-specific activity. In vitro, 4-hydroxy tamoxifen exhibits more potency than tamoxifen, as measured by binding affinity to estrogen receptors, or ERs, and a binding affinity similar to estradiol for estrogen receptors (Robertson *et al*., 1982; Kuiper *et al*., 1997).

Research data supports the use of 4-hydroxy tamoxifen for treating breast cancer. In *in vitro* studies, 4-hydroxy tamoxifen inhibits the growth of both normal and cancerous breast cells (Nomura, 1985; Malet, 1988, 2002; Charlier, 1995). Additionally, transdermally delivered 4-hydroxy tamoxifen exhibits an anti-tumor effect on human breast tumors grown subcutaneously in mice (U.S. patent No. 5,904,930). In humans, limited experiments have shown that percutaneously administered 4-hydroxy tamoxifen can concentrate in local breast tumors, with very little systemic distribution (Mauvais-Jarvis, 1986). 4-Hydroxy tamoxifen also shows promise for treating mastalgia, excessive scarring and gynecomastia, and for decreasing breast density.

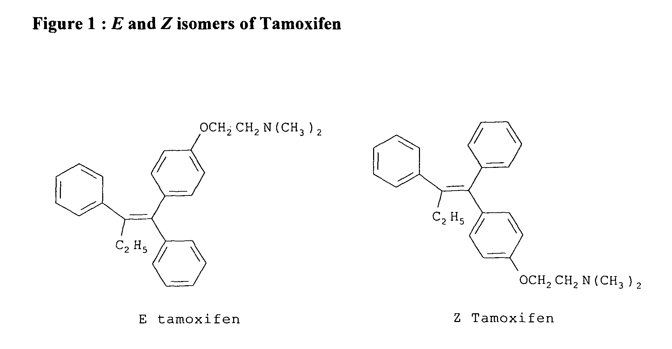

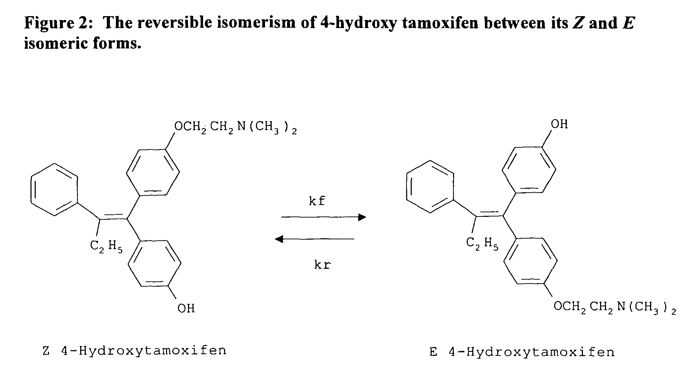

In the chemical structure of 4-hydroxy tamoxifen, or 1-[4-(2-N-dimethylaminoethoxy)phenyl]-1-(4-hydroxyphenyl)-2-phenylbut-1-ene, a double bond between two carbon atoms gives rise to two stereoisomeric forms. Unlike tamoxifen, 4-hydroxy tamoxifen does not possess two identical phenyl groups, but rather has four different groups distributed over the alkene group. Cis-trans terminology, therefore, can not properly be applied to the isomers of 4-hydroxy tamoxifen. Instead, E from the German Entgegen, meaning across, and Z from the German Zusammen, meaning together, are properly applied (see figures 1 and 2). Both isomers of 4-hydroxy tamoxifen are biologically active, but the Z isomer is more active biologically than the E isomer (U.S. Patent 6,172,263).

In the solid state, an isomeric mixture of 4-hydroxy tamoxifen is very stable. In solution, however, isomerization between the *Z* and *E* forms occurs. Malet *et al*. observed that spontaneous isomerization of *Z*- into *E*-4-hydroxy tamoxifen occurred within 24-48h, but stabilized rapidly at a Z/E ratio of 70/30, whether in stock solution, culture medium or cultured cells and regardless of temperature (-20°C, 4°C or 37°C). See Malet et al. (2002). Katzenellenbogen *et al*. further demonstrated that hydroxy tamoxifen isomers that are initially 99% pure undergo a time- and temperature-dependent isomerization, so that after 2 days in tissue culture medium at 37°C they have isomerized to the extent of 20%. This isomerization occurs more slowly at 4°C than at 37°C and its speed can be reduced by various antioxidants. See Katzenellenbogen *et al*. (1985). According to Sigma, a supplier of 4-hydroxy tamoxifen, the 4- hydroxy tamoxifen *E-Z* interconversion process is favored by solvents of low dielectric constants when exposed to light and when incubated in culture medium.

The isomerization process potentially can affect the activity of a pharmaceutical composition comprising 4-hydroxy tamoxifen as an active ingredient. To meet international pharmaceutical regulatory requirements, therefore, a need exists for chemically stable compositions of 4-hydroxy tamoxifen. A "stable" pharmaceutical composition is one whose qualitative and quantitative composition, including physical, chemical and biological characteristics, do not significantly change during time under specific conditions of temperature and moisture, *e.g*., during 3 years at 25°C/60%HR, 1 year at 30°C/65%HR and/or 6 months with 40°C/75%HR. "Significant change" refers qualitative and/or quantitative differences that might affect the potency, efficacy or safety of a pharmaceutical composition.

In providing a stable 4-hydroxy tamoxifen composition, it would be helpful to have a more complete understanding of the 4-hydroxy tamoxifen isomerization process.

**Summary of the Invention**

[The present inventors have discovered that the isomerization of 4-hydroxy tamoxifen in solution equilibrates at a Z:E isomer ratio of approximately 1:1. Moreover, they have discovered that once this equilibrated ratio is attained,

**EP 1 579 857 A1**

it remains stable.

In accord with this discovery, the present invention includes pharmaceutical compositions having 4-hydroxy tamoxifen as an active agent, wherein approximately 50% of the 4-hydroxy tamoxifen exists in *Z* isomeric form and the remainder is in *E* isomeric form. In specific embodiments, the pharmaceutical composition is formulated for percutaneous administration in a gel, a solution or another pharmaceutical form containing alcohol and an aqueous vehicle.

In a specific example, a gel formulation, the pharmaceutical compositions comprise:

    a) about 0.01 % to 0.20 % by weight of 4-hydroxy tamoxifen,

    b) about 0.5 % to 2.0 % by weight of isopropyl myristate,

    c) about 60% to 75% by weight of absolute alcohol,

    d) about 25% to 40% by weight of aqueous vehicle,

    e) about 0.5% to 5% by weight of gelling agent,

wherein the percentage of components are weight to weight of the composition.

In another aspect, the invention includes a method of treating or preventing medical conditions by administering a pharmaceutical composition having 4-hydroxy tamoxifen as an active agent, wherein approximately 50% of the 4-hydroxy tamoxifen exists in Z isomeric form and the remainder is in E isomeric form, to a patient in need thereof. Medical conditions for which such administration is useful include breast cancer, mastalgia, breast density, excessive scarring and gynecomastia.

For purposes of prophylaxis or treatment, the pharmaceutical compositions may be administered by any means that delivers 4-hydroxy tamoxifen to estrogen receptor-bearing cells *in vivo*. It is preferable that the administration be done percutaneously (topically), to avoid the first-pass effect and related liver metabolism of the 4-hydroxy tamoxifen. For percutaneous administration, 4-hydroxy tamoxifen may be applied to any skin surface. Application to the breasts is advantageous because 4-hydroxy tamoxifen tends to concentrate in local subcutaneous tissues with estrogen receptors when administered percutaneously.

A broad range of topical formulations are suitable for performing the invention, but hydroalcoholic solutions and hydroalcoholic gels are preferred. The concentration of 4-hydroxy tamoxifen in these formulations may vary, but a dose should result in local 4-hydroxy tamoxifen tissue concentrations that effectively oppose estrogenic driven effects.

In another aspect, the present invention includes a kit for storage that comprises (a) a pharmaceutical composition having 4-hydroxy tamoxifen as an active agent, wherein approximately 50% of the 4-hydroxy tamoxifen exists in Z isomeric form and the remainder is in E isomeric form, and (b) a container, wherein the pharmaceutical composition is contained within the container. In specific embodiments of this kit, the container may be a unit dose packet or a multiple dose container, such as a container with a metered pump.

## Brief Description of the Figures

    Figure 1 illustrates the *E* and *Z* isomers of tamoxifen.

    Figure 2 illustrates the reversible isomerism of 4-hydroxy tamoxifen.

    Figure 3 illustrates the isomer concentration ratio (as a percentage) for Panchim batch 98RD 10079 at 25°C.

    Figure 4 illustrates the isomer concentration ratio (as a percentage) for Panchim batch 98RD 10079 at 30°C.

    Figure 5 illustrates the isomer concentration ratio (as a percentage) for Panchim batch 98RD10079 at 40°C.

    Figure 6 illustrates the isomer concentration ratio (as a percentage) for ICI batch Bx 17 at 40°C.

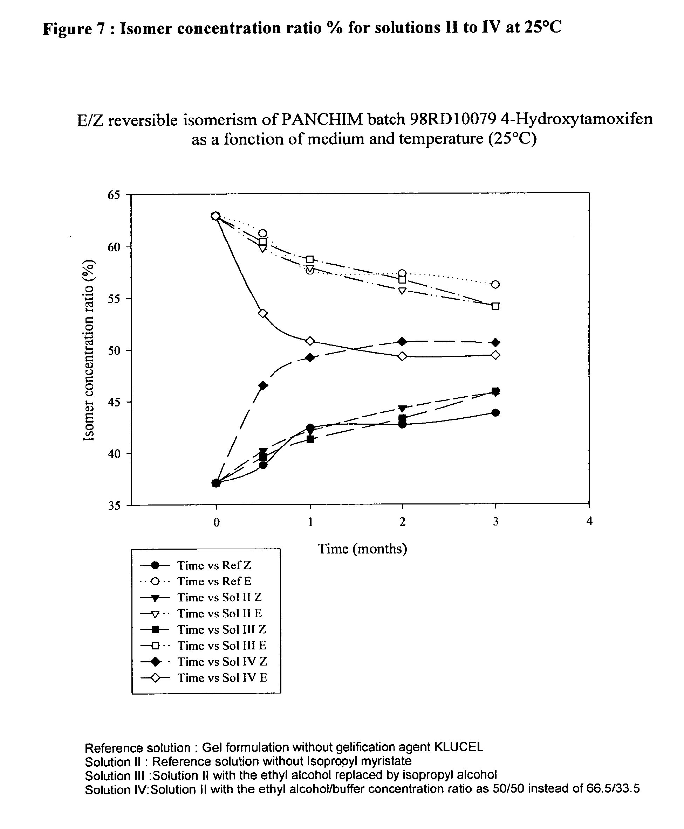

    Figure 7 illustrates the isomer concentration ratio (as a percentage) for solutions II-IV at 25°C.

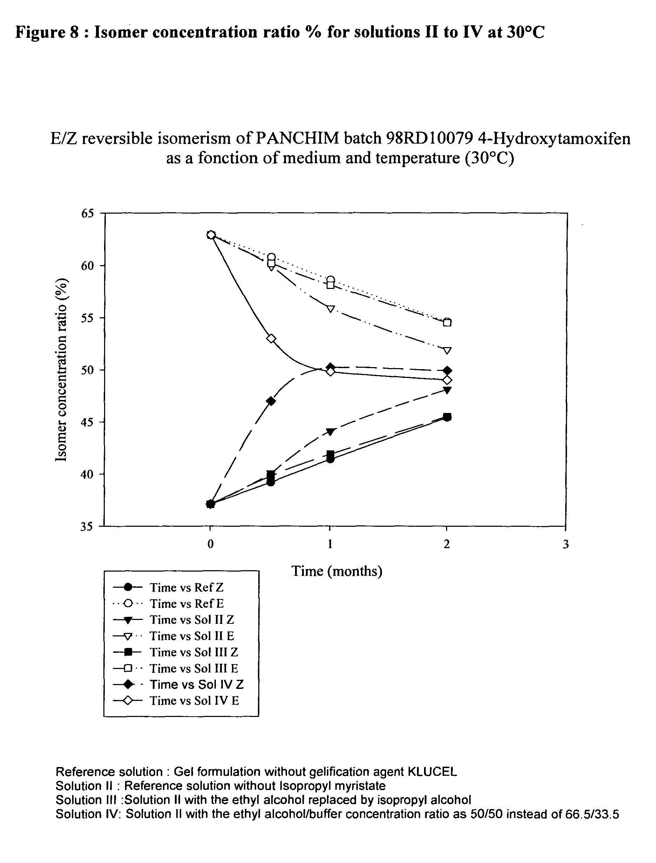

    Figure 8 illustrates the isomer concentration ratio (as a percentage) for solutions II-IV at 30°C.

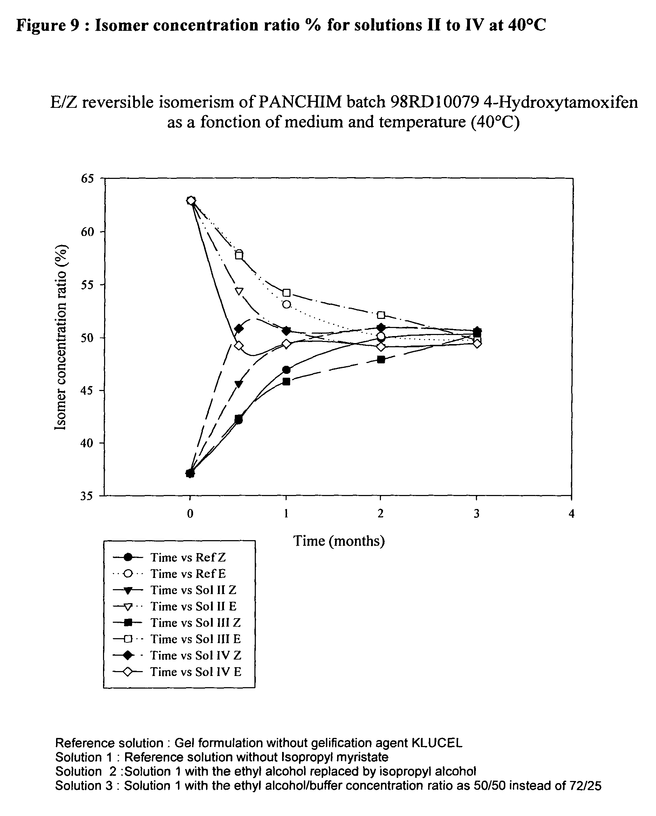

Figure 9 illustrates the isomer concentration ratio (as a percentage) for solutions II-IV at 40°C.

**Detailed Description of the Preferred Embodiments**

An important aspect of the present invention is the very surprising discovery that the isomerization of 4-hydroxy tamoxifen in solution equilibrates at a *Z:E* isomer ratio of approximately 50:50, rather than the 70:30 ratio reported by Malet *et al.*, and that once this equilibrated ratio is attained, it remains stable. The inventors further discovered that inter-conversion of the *E* and the *Z* isomers of 4-hydroxy tamoxifen (see Figure 2) is a reversible reaction having an equilibrium constant k determined by the following formula:

$$K = \frac{k_f}{k_r} = \frac{[Z]eq}{[E]eq} = 1$$

where [E] and [Z] are the equilibria concentrations of the corresponding isomers, and $k_f$ and $k_r$ respectively are the forward and reverse rate constants. The rates for the forward and reverse reactions, therefore, are equivalent.

These insights on the behavior of 4-hydroxy tamoxifen isomers make possible the development of chemically stable pharmaceutical compositions that contain roughly equal amounts of 4-hydroxy tamoxifen *Z* and *E* isomers. In such compositions, the isomerization that occurs between *Z* and *E* forms does not significantly affect the composition's potency, efficacy or safety.

The inventors have performed several experiments to study the equilibration of 4-hydroxy tamoxifen isomers under different conditions of light, temperature, pH and moisture, as well as in different media, at different concentrations of 4-hydroxy tamoxifen and at different alcohol/aqueous vehicle ratios. In brief, they prepared alcoholic solutions containing different concentrations of 4-hydroxy tamoxifen at different ratios of *Z* and *E* isomers, then observed the isomerization that occurred in those solutions over time at different temperatures and pH values (see the Examples below). By 6 months, a stable ratio (approximately 1:1 ) of *Z* and *E* isomers was attained under many conditions, and a clear trend was observable under all conditions. The rate of equilibration directly depended on temperature, pH, alcohol/aqueous vehicle content, light and 4-hydroxy tamoxifen concentration. In all cases, only the rate of equilibration was affected (see Examples below), but not the final ratio of *Z* and *E* isomers, which surprisingly remained approximately 1:1.

From a chemical kinetics viewpoint, dielectric constant is recognized as one of the fundamental properties that influences solvolytic reaction rates. In this regard, publications exist that highlight the influence of water on the degradation of molecules. For example, Sanyude *et al.*, studied the influence of the water:alcohol ratio on the degradation of aspartame. They reported that the degradation rate of aspartame increased as the dielectric constant of the solvent medium decreased, *i.e.*, when the water concentration in the medium decreases. By contrast, the present inventors have found that the isomerization rate of 4-hydroxy tamoxifen is increased as the dielectric constant of the solvent medium is increased, *i.e.*, when the water concentration in the medium increases.

In accord with the inventors' discoveries, the present invention includes pharmaceutical compositions that comprise 4-hydroxy tamoxifen, wherein about 50% of the 4-hydroxy tamoxifen exists in a *Z* isomeric form and the remainder of the 4-hydroxy tamoxifen exists in an *E* isomeric form. In specific embodiments, about 45% - 55%, about 46% - 54%, about 47% - 53%, about 48% - 52%, about 49% - 51% or about 50% of the 4-hydroxy tamoxifen is in a *Z* isomeric form. Preferably about 49% - 51 %, and more preferably about 50% of the 4-hydroxy tamoxifen is in a *Z* isomeric form. These contents are defined at the equilibrium state and not at the manufacture of the pharmaceutical composition.

Equilibrated ratios of *Z* and *E* 4-hydroxy tamoxifen isomers can be obtained in a pure alcoholic composition or a mixture of an alcohol and a aqueous vehicle by admixing known quantities of the isomers or by subjecting the composition to conditions that speed the equilibration process, such as high temperature, high 4-hydroxy tamoxifen content, high aqueous vehicle content or UV light. The inventors have shown that the molecular size of the alcohol (ethanol or isopropanol) does not have an effect on the rate of isomerization.

Pharmaceutical compositions of the present invention may be formulated in any dosage form capable of delivering 4-hydroxy tamoxifen to estrogen receptors *in vivo.* Preferably, the compositions are formulated for "percutaneous administration," a phrase that denotes any mode of delivering a drug from the surface of a patient's skin, through the stratum corneum, epidermis, and dermis layers, and into the microcirculation. This is typically accomplished by diffusion down a concentration gradient. The diffusion may occur via intracellular penetration (through the cells), intercellular penetration (between the cells), transappendageal penetration (through the hair follicles, sweat, and sebaceous glands), or any combination of these.

Percutaneous administration of 4-hydroxy tamoxifen offers several advantages. First, it avoids the hepatic metabolism that occurs subsequent to oral administration (Mauvais-Jarvis *et al.*, 1986). Second, percutaneous administration significantly reduces systemic drug exposure, and the attendant risks from non-specifically activating estrogen receptors throughout the body; this, because topical 4-hydroxy tamoxifen is absorbed primarily into local tissues. In particular, when 4-hydroxy tamoxifen is percutaneously applied to breasts, high concentrations accumulate in the breast tissue, presumably due to many estrogen receptors therein, without creating a high plasma concentration (Mauvais-Jarvis *et al.*, *supra*).

The effectiveness of percutaneous drug administration depends on many factors, including drug concentration, surface area of application, time and duration of application, skin hydration, physiochemical properties of the drug, and partitioning of the drug between the formulation and the skin. Drug formulations intended for percutaneous use take advantage of these factors to achieve optimal delivery. Such formulations often comprise penetration enhancers that improve percutaneous absorption by reducing the resistance of the stratum corneum by reversibly altering its physiochemical properties, changing hydration in the stratum corneum, acting as cosolvent, or changing the organization of lipids and proteins in the intercellular spaces. Such enhancers of percutaneous absorption include surfactants, DMSO, alcohol, acetone, propyleneglycol, polyethylene glycol, fatty acids or fatty alcohols and their derivatives, hydroxyacids, pyrrolidones, urea, essential oils, and mixtures thereof. In addition to chemical enhancers, physical methods can increase percutaneous absorption. For example, occlusive bandages induce hydration of the skin. Other physical methods include iontophoresis and sonophoresis, which use electrical fields and high-frequency ultrasound, respectively, to enhance absorption of drugs that are poorly absorbed due to their size and ionic characteristics.

The many factors and methods relating to percutaneous drug delivery are reviewed in REMINGTON: THE SCIENCE AND PRACTICE OF PHARMACY, Alfonso R. Gennaro (Lippincott Williams & Wilkins, 2000), at pages 836-58, and in PERCUTANEOUS ABSORPTION: DRUGS COSMETICS MECHANISMS METHODOLOGY, Bronaugh and Maibach (Marcel Dekker, 1999). As these publications evidence, those in the pharmaceutical field can manipulate the various factors and methods to achieve efficacious percutaneous delivery.

For percutaneous administration, 4-hydroxy tamoxifen may be delivered in a hydroalcoholic solution, hydroalcoholic gel, ointment, cream, gel, emulsion (lotion), powder, oil or similar formulation.

In preferred embodiments of the invention, 4-hydroxy tamoxifen is formulated in a alcoholic formulation, preferably in a hydroalcoholic gel. The amount of 4-hydroxy tamoxifen in such a gel may range from about 0.001 to about 1.0 gram of 4-hydroxy tamoxifen per 100 grams of gel. Preferably, it ranges from about 0.01 to about 0.2 gram of 4-hydroxy tamoxifen per 100 grams of gel. In such embodiments, 4-hydroxy tamoxifen may constitute about 0.01%, 0.02%, 0.03%, 0.04%, 0.05%, 0.06%, 0.07%, 0.08%, 0.09%, 0.10%, 0.11 %, 0.12%, 0.13%, 0.14%, 0.15%, 0.16%, 0.17%, 0.18%, 0.19% or 0.20% by weight of the pharmaceutical composition.

4-Hydroxy tamoxifen formulations of the invention generally will comprise one or more nonaqueous vehicles, such as alcoholic vehicles. These vehicles should be capable of dissolving both 4-hydroxy tamoxifen and any penetration enhancer used. They also should have a low boiling point, preferably less than 100°C at atmospheric pressure, to permit rapid evaporation upon contact with the skin. Preferred alcoholic vehicles are ethanol and isopropanol. In particular, ethanol effectively contributes to the percutaneous absorption of 4-hydroxy tamoxifen by rapidly evaporating upon contact with skin. The amount of absolute alcoholic vehicle in a formulation according to the invention generally ranges between 35% and 99.9%, preferably between 50% and 85%, more preferably between 60% and 75% by weight. Thus, the amount of absolute nonaqueous vehicle in a gel formulation may be about 60%, 61%, 62%, 63%, 64%, 65%, 66%, 67%, 68%, 69%, 70%, 71%, 72%, 73%, 74% or 75% by weight.

Formulations also may comprise an aqueous vehicle, which permits solubilization of any hydrophilic molecules in a formulation, and also promotes moisturization of the skin. An aqueous vehicle also can regulate pH, preferably in the range of about 4 to about 12, more preferably in the range of about 6 to about 11, even more preferably in the range of about 8 to about 10, and most preferably at about 9. As shown below, the pH, and therefore the choice of a buffer solution, affects the rate of equilibration between 4-hydroxy tamoxifen *E* and *Z* isomers. The final equilibrium ratio, however, remains equal to about 1:1 regardless of the buffer.

Aqueous vehicles include alkalinizing and basic buffer solutions, including phosphate buffered solutions (e. g., dibasic or monobasic sodium phosphate), citrate buffered solutions (e.g., sodium citrate or potassium citrate) and simply purified water. The phosphate buffer is preferred according to the invention. The amount of an aqueous vehicle preferably ranges between 0.1% and 65% by weight of the pharmaceutical composition, more preferably between 15% and 50%, and still more preferably between 25% and 40%. Thus, the amount of an aqueous vehicle may be about 25%, 26%, 27%, 28%, 29%, 30%, 31%, 32%, 33%, 34%, 35%, 36%, 37%, 38%, 39% or 40%. In the case that formulations contain an aqueous vehicle, the amount of absolute alcoholic vehicle in a formulation is preferably from about 60% to about 75%.

4-Hydroxy tamoxifen formulations may also comprise one or more percutaneous absorption enhancers. The preferred percutaneous absorption enhancers are fatty acid esters. One highly preferred example of a fatty acid ester penetration enhancer is isopropyl myristate. When isopropyl myristate is used in a gel, the amount may range from about 0.1 to about 5.0 grams per 100 grams of gel. Preferably, the amount of isopropy myristate ranges from about 0.5 to about 2.0 grams per 100 grams of gel. In such embodiments, isopropyl myristate may constitute about 0.5%, 0.6%, 0.7%, 0.8%, 0.9%, 1.0%, 1.1 %, 1.2%, 1.3%, 1.4%, 1.5%, 1.6%, 1.7%, 1.8%, 1.9% or 2.0% by weight of the pharmaceutical composition.

Additionally, 4-hydroxy tamoxifen formulations may comprise one or more gelling agents to increase the viscosity of a formulation and/or to function as a solubilizing agent. Depending on the gelling agent's nature, it may constitute between 0.1 % and 20% by weight of a formulation, preferably between 0.5% and 10%, more preferably between 0.5% and 5%. Thus, the amount of a gelling agent may be about 0.5%, 1.0%, 1.5%, 2.0%, 2.5%, 3.0%, 3.5%, 4.0%, 4.5% or 5.0%. Preferred gelling agents include carbomers, cellulose derivatives, poloxamers and poloxamines. More particularly, preferred gelling agents are chitosan, dextran, pectins, natural gum and cellulose derivatives such as ethyl cellulose, hydroxypropyl cellulose, hydroxyethyl cellulose, hydroxypropyl methyl cellulose (HPMC), carboxymethyl cellulose (CMC), and the like. One highly preferred gelling agent is hydroxypropyl cellulose.

When a formulation comprises a gelling agent, in particular a non-preneutralized acrylic polymer, it may advantageously also comprise a neutralizing agent. The neutralizing agent/gelling agent ratio preferably is between 10:1 and 0.1:1, more preferably between 7:1 and 0.5:1, and still more preferably between 4:1 and 1:1. Thus, the neutralizing agent/gelling agent ratio may be about 7:1, 6:1, 5:1, 4:1, 3:1, 2:1, 1:1 or 0.5:1. A neutralizing agent should form, in the presence of the polymer, salts that are soluble in the vehicle. A neutralizing agent also should permit optimum swelling of polymer chains during neutralization of charges and formation of polymer salts. Useful neutralizing agents include sodium hydroxide, ammonium hydroxide, potassium hydroxide, arginine, aminomethylpropanol, trolamine and tromethamine. Those skilled in the art will select a neutralizing agent according to the type of gelling agent employed in a formulation. When cellulose derivatives are used as gelling agents, however, no neutralizing agents are required.

Table 1 describes the composition of two highly preferred 4-hydroxy tamoxifen gel formulations. All the component are pharmaceutically acceptable components.

### Table 1: Composition of 4-Hydroxy Tamoxifen Gel Formulations

| Ingredient | Quantity per 100 g of gel | |
| --- | --- | --- |
| | 20 mg 4-OHT Gel | 57 mg 4-OHT Gel |
| 4-Hydroxy Tamoxifen | 0.02 g | 0.057 g |
| Absolute Ethyl Alcohol | 66.5 g | 66.5 g |
| Isopropyl myristate | 1 g | 1 g |
| Hydroxypropylcellulose | 1.5 g | 1.5 g |
| Phosphate Buffer (pH 7, diluted 1:4) | q.s. 100 g | q.s. 100 g |

Pharmaceutical compositions of the invention may be administered to treat numerous medical conditions for which tamoxifen and 4-hydroxy tamoxifen are useful. For example, they may be administered to treat breast cancer (Mauvais-Jarvis, 1986; Example 4), mastalgia (Fentiman 1986, 1988, 1989), excessive scarring (Hu, 1998; Hu 2002) or gynecomastia (Gruntmanis and Braunstein (2001)). They also may be administered to prevent breast cancer in patients at high risk for developing that disease or to reduce breast density when that condition interferes with mammography (Atkinson, 1999; Brisson, 2000; Son, 1999). See also U.S. provisional patent application Nos. 60/433,959, filed December 18, 2002; 60/433,958, filed December 18, 2002; and 60/458,963, filed April 1, 2003, each of which is incorporated herein by reference, for a complete description of these uses.

Although the invention is not constrained to any particular theory, clinically significant side effects of anti-estrogen agents occur when the agents displace estradiol in non-target tissues. Because 4-hydroxy tamoxifen and estradiol have similar binding affinities for estrogen receptors, a competition between them for receptor binding would be approximately equal when the concentration of each compound approximates that of the other. If the 4-hydroxy tamoxifen concentration exceeds the estradiol concentration, the former will be bound preferentially to the estrogen receptors, and vice versa. By administering 4-hydroxy tamoxifen locally, high concentrations can be achieved in the target tissues without simultaneously raising 4-hydroxy tamoxifen plasma levels to a point where significant systemic competition for estradiol receptors occurs.

In women, doses of 4-hydroxy tamoxifen that result in plasma concentrations less than about 80 pg/mL, or the mean estradiol concentration in normal premenopausal women, are preferred. More preferably, doses of 4-hydroxy tamoxifen will result in plasma concentrations less than about 50 pg/mL. In men, doses of 4-hydroxy tamoxifen that result in plasma concentrations less than about 20 pg/mL, or the mean estradiol concentration in normal men, are preferred. The daily doses to be administered can initially be estimated based upon the absorption coefficients of 4-hydroxy tamoxifen, the breast tissue concentration that is desired, and the plasma concentration that should not be exceeded. Of course, the initial dose may be optimized in each patient, depending on individual responses.

When administering a percutaneous formulation for breast conditions, doses on the order of 0.25-2.0 mg/breast/day of 4-hydroxy tamoxifen should achieve the desired result, with doses of about 0.5-1.0 mg/breast/day being preferred. In particular embodiments, the dosage is about 0.5, 0.75 or 1.0 mg/breast/day of 4-hydroxy tamoxifen.

For the treatment of excessive scarring, doses on the order of 0.25 to 6 $\mu$g of 4-hydroxy tamoxifen/cm$^2$/day should achieve the desired result, with doses of about 0.25 to 3 $\mu$g being preferred, and doses of 0.5 to 2.5 $\mu$g/cm$^2$/day being more preferred. Doses of about 1.0 and 2.0 $\mu$g/cm$^2$/day are highly preferred for treating scarring conditions.

Pharmaceutical compositions of the invention may be packaged into kits for storage. Such kits comprise (a) a pharmaceutical composition as described herein, and (b) a container, wherein the pharmaceutical composition is contained within the container. The container may be a unit dose packet, such as a foil packet, or a multiple dose container, such as a container with a metered pump. Preferably, the container is impervious to light.

Reference to the following, illustrative examples will help to provide a more complete understanding of the invention.

## **Example 1**

This example demonstrates that isomerization of 4-hydroxy tamoxifen occurs in solution and that the isomerization ultimately reaches an equilibrium at which about 50% of the 4-hydroxy tamoxifen exists in the Z isomeric form, with the remainder being in the E isomeric form.

A. Preparation of Solutions Containing 4-Hydroxy tamoxifen

Hydroalcoholic solutions containing 4-hydroxy tamoxifen were prepared, based on the following gel formula:

| | |
|---|---|
| 4-hydroxy tamoxifen | 0.057 g |
| Isopropyl myristate | 1.000 g |
| Klucel | 1.500 g |
| Absolute ethanol | 66.500 g |
| buffer phosphate qsf | 100.000 g |

In the hydroalcoholic solutions, phosphate buffer was substituted for Klucel. The composition of the buffer was as follows:

| | |
|---|---|
| $KH_2PO_4$ | 0.8526 g |
| $Na_2HPO_4$ | 3.4826 g |
| Purified water | 1000 g |

Solutions containing five different concentrations (0.02%, 0.04%, 0.06%, 0.08% and 0.10%) of 4-hydroxy tamoxifen were prepared. The composition of each solution is shown in the table below.

| | 0.02 % 4-OHT | 0.04% 4-OHT | 0.06% 4-OHT | 0.08% 4-OHT | 0.10% 4-OHT |
|---|---|---|---|---|---|
| 4-OHT (g) | 0.016 | 0.032 | 0.048 | 0.064 | 0.080 |
| Absolute Ethanol (g) | 53.210 | 53.210 | 53.210 | 53.210 | 53.210 |
| Myristate (g) | 0.800 | 0.800 | 0.800 | 0.800 | 0.800 |
| buffer qsf (g) | 80.000 | 80.000 | 80.000 | 80.000 | 80.000 |

At each of the stated concentrations, separate solutions containing Z-4-hydroxy tamoxifen or a mixture of *Z-*

and *E*-4-hydroxy tamoxifen were prepared. 4-hydroxy tamoxifen from three separate manufacturers was tested:

- PANCHIM : 4-OHT *E+Z* (batch 98RD 10079)
- PANCHIM : 4-OHT *Z* (batch 7421)
- ICI :    4-OHT *Z* (batch Bx 17)
- SIGMA:    4-OHT *Z* (batch 092K4075).

Because only a small quantity of Z-4-hydroxy tamoxifen was available from SIGMA, only a 0.06% solution of that batch was prepared. This solution was tested, as described below, only at 25°C and 40°C.

B. Study Conditions

Each solution was subdivided into three parts in 30 ml brown glass bottles, then placed in ovens regulated at 25°, 30°, and 40°C.

The relative amounts of *Z*- and *E*-4-hydroxy tamoxifen isomers were determined at the initiation of the study and at 2-week, 1-month, 2-month, 3-month, 4-month and 5-month time points.

C. Analytical Methods

HPLC was employed to determine the relative amounts of each 4-hydroxy tamoxifen isomer, using a standard solution of 4 hydroxy tamoxifen as a reference. Operating parameters for the HPLC were as follows:

| Column | BECKMAN ULTRASPHÈRE ODS 250x4.6 mm 5µm |
|---|---|
| Mobile phase | 60 % a 0.5% aqueous solution of triethylamine adjusted to pH 2.5 with 25% hydrochloric acid 40 % acetonitrile |
| Flow rate | 0.8 ml/min |
| Wavelength | 245 nm |
| Volume of injection | 20 µl |
| Run time | 20 min |

For HPLC, the hydro alcoholic solutions were diluted in the mobile phase to obtain a 4-hydroxy tamoxifen concentration close to 2.3 µg/ml.

| Initial concentration | Volume to take | Mobile phase qsp |
|---|---|---|
| 0.02 % | 645 µl | 50 ml |
| 0.04 % | 325 µl | 50 ml |
| 0.06 % | 215 µl | 50 ml |
| 0.08 % | 160 µl | 50 ml |
| 0.10 % | 130 µl | 50 ml |

The order of elution was as follows:

4-OHT E : retention time about 13.3 min
4-OHT Z : retention time about 15.0 min.

Percentages of each isomer were calculated using the following formulas:

$$\% \text{ isomer E} = \frac{\text{OHT E peak area}}{\text{OHT } (E{+}Z) \text{ peak area}} \times 100$$

$$\% \text{ isomer Z} = \frac{\text{OHT Z peak area}}{\text{OHT } (E{+}Z) \text{ peak area}} \times 100$$

D. Data fitting :

The reversible isomerization reaction and the equilibrium reaches are lied by the following relationship :

$$\log \frac{A_o - A_{eq}}{A - A_{eq}} = \frac{k_f + k_r}{2.303} xt$$

where

A$_o$ = initial concentration of the reagent
A$_{eq}$ = concentration of the same reagent at equilibrium
A = the concentration of A at time t
k$_f$ = forward rate constant
k$_r$ = reverse rate constant
t = time measured in months

A plot of $\log \frac{A_o - A_{eq}}{A - A_{eq}}$ as a function of time gives a straight line fit with a slope of $\frac{k_f + k_r}{2.303}$ and a y-intercept of 0.

E. Results

The tables below show results for the study; by the 6-month time point, many solutions had equilibrated at approximately a 1:1 ratio of *Z* and *E* isomers. The rate constant of the isomer inter-conversion directly depended on both temperature, initial concentration of the pure *Z* isomer and the initial concentration of the *E/Z* isomer mixture.

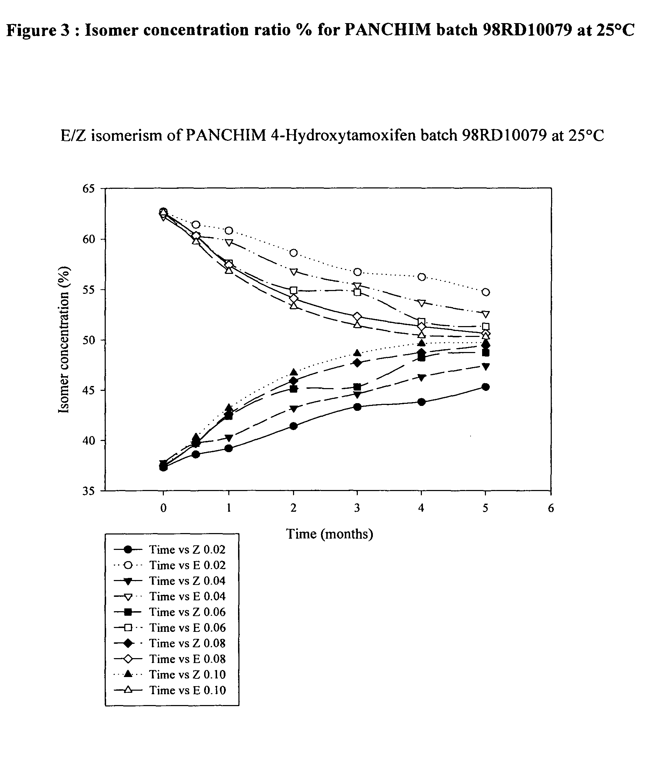

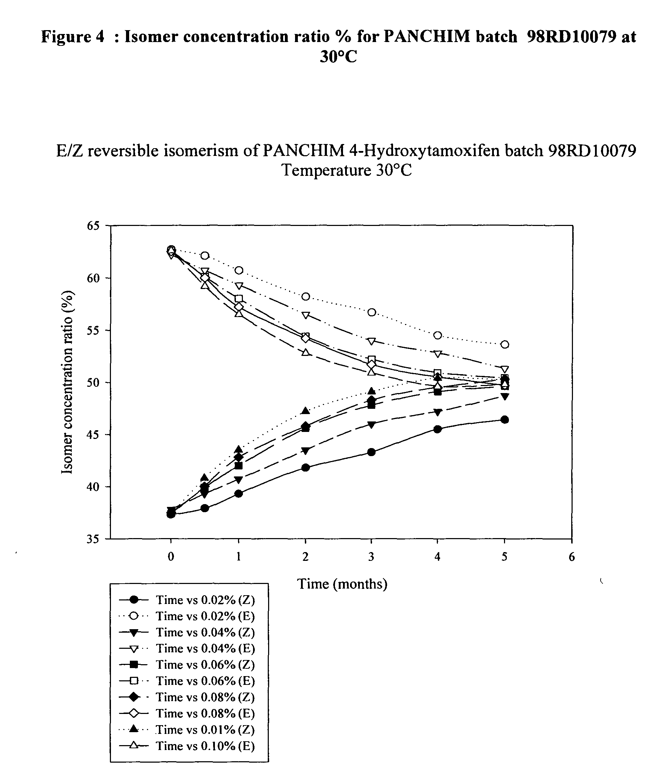

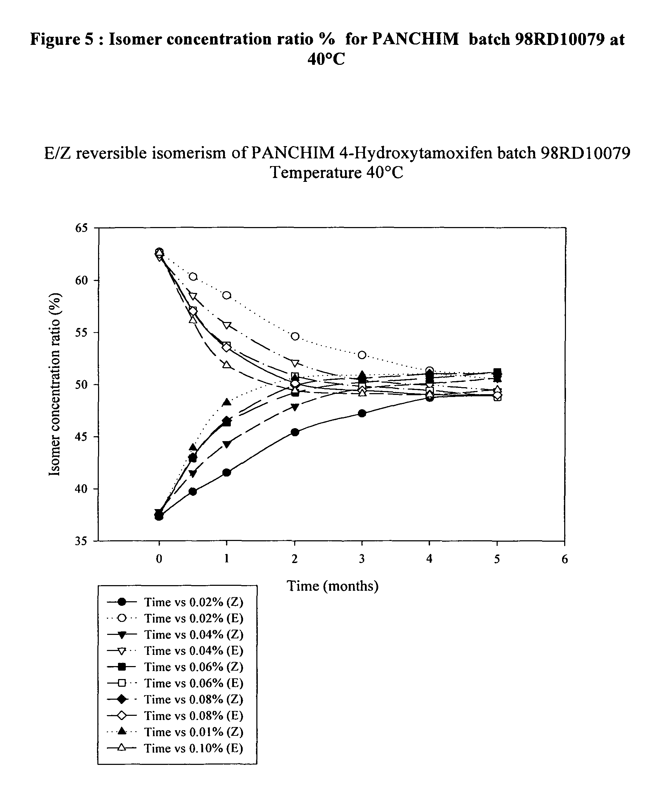

The individual *Z* and *E* isomer content as a function of time are presented in figures 3, 4 and 5 for 4-hydroxy tamoxifen over the concentration range of 0.02% to 0.1% total. 4-Hydroxy tamoxifen solution was stored at 25°C (figures 3), 30°C (figure 4) and 40°C (figure 5). The 4-hydroxy tamoxifen drug substance used to prepare the solution had an initial concentration ratio for *E* and *Z* isomers of 63 % and 37 %, respectively (PANCHIM batch 98RD 10079).

As the nominal 4-hydroxy tamoxifen was varied from 0.02% to 0.10%, the magnitude of the rate constants for the reversible isomerism increased linearly. The magnitude of the rate constants also increased with temperature.

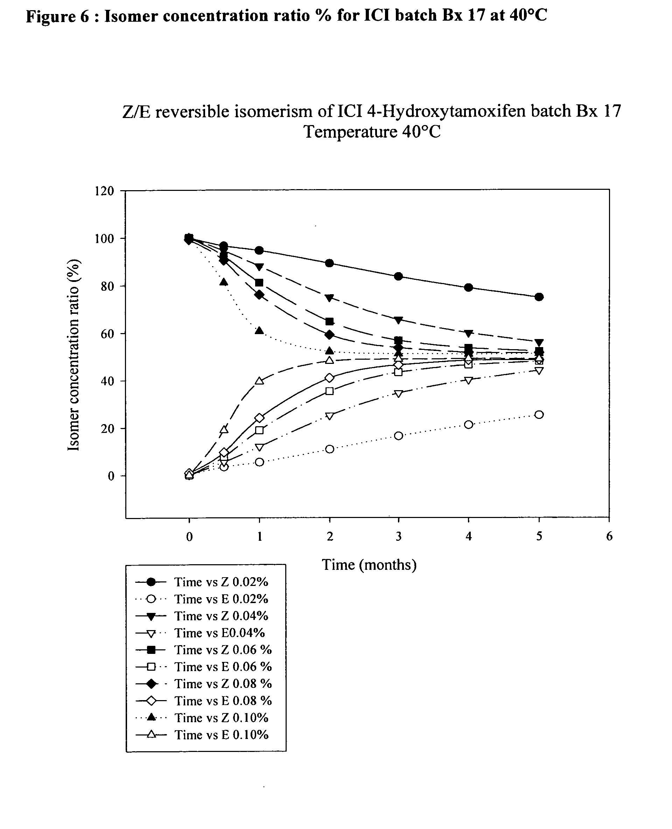

The same equilibrium ratio at approximately a 1:1 was observed starting from a 4-hydroxy tamoxifen drug substance with an initial *E/Z* ratio of 2/98 (Batches PANCHIM 7421 ) and 0/100 (batch ICI Bx 17) (see figure 6 for example). Batches PANCHIM 7421 (*E/Z* ratio 2/98) and ICI Bx 17 (*E/Z* ratio 0/100) presented rate constants that were very similar in magnitude for each nominal 4-hydroxy tamoxifen concentration and at each temperature investigated. Surprisingly, the closer the initial *E/Z* ratio is to 1:1, the higher the rate constant to reach the equilibrium is (see table 1).

## Table 1 : Rate constants

Batch PANCHIM 98RD10079 (Initial *E/Z* ratio : 62.5/37.5)

| Rate constant (month$^{-1}$)for a nominal 4-hydroxy tamoxifen concentration $(Z+E)$ in solution (%) | | | | |
|---|---|---|---|---|
| 0.02 | 0.04 | 0.06 | 0.08 | 0.10 |
| 25°C | 0.0864 | 0.2556 | 0.1769 | 0.2179 | 0.254 |
| 30°C | 0.1139 | 0.1726 | 0.2374 | 0.2932 | 0.3231 |
| 40°C | 0.2192 | 0.3761 | 0.4741 | 0.6026 | 0.8472 |

Batch PANCHIM 7421 (Initial *E/Z* ratio : 2/98)

| Rate constant (month$^{-1}$)for a nominal 4-hydroxy tamoxifen concentration $(Z+E)$ in solution (%) | | | | |
|---|---|---|---|---|
| 0.02 | 0.04 | 0.06 | 0.08 | 0.10 |
| 25°C | 0.0225 | 0.0637 | 0.0886 | 0.1352 | 0.1827 |
| 30°C | 0.0457 | 0.0864 | 0.1487 | 0.1900 | 0.2568 |
| 40°C | 0.1097 | 0.2287 | 0.3877 | 0.5311 | 0.6300 |

ICI batch Bx 17 (Initial *E/Z* ratio : 0/100)

| Rate constant (month$^{-1}$)for a nominal 4-hydroxy tamoxifen concentration $(Z+E)$ in solution (%) | | | | |
|---|---|---|---|---|
| 0.02 | 0.04 | 0.06 | 0.08 | 0.10 |
| 25°C | 0.0347 | 0.0484 | 0.0916 | 0.1476 | 0.2169 |
| 30°C | 0.0254 | 0.0801 | 0.1563 | 0.2718 | 0.3103 |
| 40°C | 0.0722 | 0.2323 | 0.3924 | 0.5095 | 0.6960 |

### Example 2:

This example demonstrates the effects of isopropyl myristate, the nature of the alcohol present, and the alcohol/phosphate buffer ratio on the isomerization of 4-hydroxy tamoxifen. It appears that the choice of a non-aqueous vehicle (buffer or water) does not significantly affect the isomerization process. The amount of the non-aqueous vehicle is indirectly proportional to the rate of isomerization, but the end $Z/E$ equilibrium ratio was unaffected by the amount of non-aqueous vehicle.

Solutions containing 0.06 % 4-hydroxy tamoxifen were prepared as described in the table below :

|  | Reference | Solution II | Solution III | Solution IV |
|---|---|---|---|---|
| 4-OHT $E+Z$ (g) | 0.048 | 0.048 | 0.048 | 0.048 |
| Myristate (g) | 0.800 | / | / | / |
| Alcohol (g) | Absolute ethanol 53.210 | Absolute ethanol 53.210 | Isopropanol 53.210 | Absolute ethanol 40.000 |
| buffer Qs (g) | 80.000 | 80.000 | 80.000 | 80.000 |

Solutions II, III and IV described above were prepared with PANCHIM batch 98RD 10079 of 4-hydroxy tamoxifen. The initial $E/Z$ ratio was 63%/37%.

Reference solution : Gel formulation without the gelling agent KLUCEL

Solution II : Reference solution without Isopropyl myristate and containing ethanol
Solution III : Solution II with the ethanol replaced by Isopropyl alcohol
Solution IV : Solution II with the ethanol/ buffer ratio of 50/50 instead of 66.5/33.5

As in Example 1, isomerization in each solution was tracked over time. The figures below show results.

The effect of replacing ethanol by isopropyl alcohol and eliminating the isopropyl myristate have no significant effect on the reversible kinetics of 4-hydroxy tamoxifen.

Increasing the aqueous vehicle (buffer) concentration from 33.5% to 50% greatly increases the rate constant at 25°C and 30°C. The difference is less pronounced at 40°C. The increase in buffer concentration increase the electric constant of the mixture and thus facilitates the polarization of the hydroxyl group and consequently the conjugation of the double bond of the alkene group of 4-hydroxy tamoxifen. The same phenomenon was observed replacing buffer with water. It is important to note also the role of the pH of the buffer solution on the kinetics of 4-hydroxy tamoxifen reversible isomerism.

### Example 3:

This example demonstrates the effects of extreme temperature on 4-hydroxy tamoxifen isomerization. Extremely high temperatures speed the equilibration process, while extremely low temperatures slow it. This example also shows that the amount of aqueous vehicle contained in the mixture affects the rate of isomerization, as noted in Example 2.

Two solutions containing 0.06% $Z$-4-hydroxy tamoxifen were prepared: solution V and solution VI. Solution V contained 4-hydroxy tamoxifen in pure ethanol solution. Solution VI contained 4-hydroxy tamoxifen in a mixture of 66.3% water and 33.7% ethanol. The amount of isomerization that occurred in each solution was observed after one week at -20 °C, 25 °C and 60 °C. The table below shows results. After one week, no isomerization was observed at -20 °C and 25 °C, whereas a beginning of isomerization was observable and 60 °C. In view of the results published by Malet *et al.*, it was surprising that isomerization was not detectable in solutions V and VI after a week of storage at -20°C and 25°C.

Table 2:

| Effects of Extreme Temperature and Alcohol Content on Isomerization | | | |
|---|---|---|---|
| **-20 °C** | | **Solution V** | **Solution VI** |
| **T0** | % E | 0 | 0 |
| | % Z | 100 | 100 |
| **1 week** | % E | 0 | 0 |
| | % Z | 100 | 100 |
| | | | |
| | | | |
| **25 °C** | | **Solution V** | **Solution VI** |
| **T0** | % E | 0 | 0 |
| | % Z | 100 | 100 |
| **1 week** | % E | 0 | 0 |
| | % Z | 100 | 100 |
| | | | |
| | | | |
| **60 °C** | | **Solution V** | **Solution VI** |
| **T0** | % E | 0 | 0 |
| | % Z | 100 | 100 |
| **1 week** | % E | 19.0 | 50.6 |
| | % Z | 81.0 | 49.4 |

### Example 4:

This example demonstrates the effects of light on 4-hydroxy tamoxifen isomerization. The amount of isomerization that occurred in each solution containing 0.06% of 4-hydroxy tamoxifen with an initial $E/Z$ ratio of 98/2 (in a mixture of 66% water and 34% ethanol) was observed during 2 hours at room temperature and at 2 UV wavelengths: 380 nm and 254 nm. Table 3 below shows results.

UV light speeds the equilibration process; indeed, in 5 min it is possible to observe the isomerization process at room temperature. At 254 nm, the isomerization process is faster than at 380 nm. Also at 254 nm, the phenomenon of isomerization combines with the degradation of the two isomers into impurities called imp1 and imp2, which are phenanthrenes derivatives. Thus the $E/Z$ isomer ratio equilibrium reached under UV light is different from 1/1.

Table 3:

| Effects of Light on Isomerization | | | | | | | | | | |
|---|---|---|---|---|---|---|---|---|---|---|
| **380 nm** | **T0** | **5 min** | **10 min** | **15 min** | **30 min** | **45 min** | **1 h** | **1 h 15** | **1 h 30** | **1 h 45** | **2 h** |
| % E | 1.6 | 1.6 | 1.7 | 1.6 | 1.8 | 1.9 | 2.0 | 2.8 | 2.9 | 2.9 | 3.3 |
| % Z | 98.4 | 98.4 | 98.3 | 98.4 | 98.2 | 98.1 | 98.0 | 97.2 | 97.1 | 97.1 | 96.7 |
| **254 nm** | **T0** | **5 min** | **10 min** | **15 min** | **30 min** | **45 min** | **1 h** | **1 h 15** | **1 h 30** | **1 h 45** | **2 h** |
| % E | 1.7 | 3.0 | 3.1 | 3.5 | 5.7 | 6.7 | 7.2 | 7.5 | 8.2 | 8.2 | 8.3 |
| % Z | 98.3 | 91.5 | 90.5 | 86.5 | 74.4 | 65.1 | 56.6 | 52.7 | 43.5 | 38.2 | 32.5 |
| % imp 1 | 0.0 | 0.0 | 0.0 | 1.3 | 4.3 | 7.8 | 10.2 | 11.4 | 14.0 | 16.5 | 18.2 |
| % imp 2 | 0.0 | 5.5 | 6.3 | 8.7 | 15.6 | 20.4 | 26.0 | 28.4 | 34.2 | 37.1 | 41.0 |

**Example 5:**

This example demonstrates the effects of pH on 4-hydroxy tamoxifen isomerization. The amount of isomerization that occurred in solutions containing 0.06% of 4-hydroxy tamoxifen with initial E/Z ratios of 100/0 or 37/63 was observed for two months at 40°C. The 4-hydroxy tamoxifen was in a mixture of 66.5 % absolute ethanol, 32.4% of either phosphate (pH 2 to 8) or carbonate (pH 10) buffer, and 1% of isopropyl myristate. Table 4, below, shows results.

Table 4:

| Effects of pH on Isomerization at 40°C | | | | | | | | |
|---|---|---|---|---|---|---|---|---|
| **Initial E/Z ratios of 100/0** | | | Time 0 | Day 8 | Day | Mo. 1 | Mo. 2 | Mo.3 |
| Buffer pH 2.2 | Final pH 4 | % E | 0.0 | 29.0 | 45.9 | 47.4 | 48.0 | 47.7 |
| | | % Z | 100.0 | 71.0 | 54.1 | 52.6 | 52.0 | 52.3 |
| Buffer pH 4 | Final pH 6 | % E | 0.0 | 12.5 | 34.3 | 43.2 | 48.1 | 48.5 |
| | | % Z | 100.0 | 87.5 | 65.7 | 56.8 | 51.9 | 51.5 |
| Buffer pH 6 | Final pH 7 | % E | 0.0 | 2.9 | 9.6 | 16.6 | 31.3 | 40.8 |
| | | % Z | 100.0 | 97.1 | 90.4 | 83.4 | 68.6 | 59.2 |
| Buffer pH 8 | Final pH 9 | % E | 0.0 | 3.4 | 11.6 | 20.6 | 36.4 | 44.1 |
| | | % Z | 100.0 | 96.6 | 88.4 | 79.4 | 63.6 | 55.9 |
| Buffer pH 10 | Final pH 12 | % E | 0.0 | 48.3 | 48.8 | 48.8 | 48.8 | 48.9 |
| | | %Z | 100.0 | 51.7 | 51.2 | 51.2 | 51.2 | 51.1 |
| **Initial E/Z ratios of 37/63** | | | Time 0 | Day 8 | Day | Mo. 1 | Mo. 2 | Mo.3 |
| Buffer pH 2.2 | Final pH 4 | % E | 62.9 | 52.5 | 48.5 | 48.2 | 48.0 | 48.0 |
| | | % Z | 37.1 | 47.5 | 51.5 | 51.8 | 52.0 | 52.0 |
| Buffer pH 4 | Final pH 6 | % E | 62.9 | 56.8 | 51.0 | 49.4 | 49.0 | 48.5 |
| | | % Z | 37.1 | 43.2 | 49.0 | 50.6 | 51.0 | 51.5 |
| Buffer pH 6 | Final pH 7 | % E | 62.5 | 61.1 | 58.0 | 55.8 | 52.0 | 50.4 |
| | | % Z | 37.5 | 38.9 | 42.0 | 44.2 | 48.0 | 49.6 |
| Buffer pH 8 | Final pH 9 | % E | 62.7 | 60.6 | 56.9 | 54.2 | 50.8 | 49.9 |
| | | % Z | 37.3 | 39.4 | 43.1 | 45.8 | 49.2 | 50.1 |
| Buffer pH 10 | Final pH 12 | % E | 63.4 | 48.5 | 49.0 | 49.0 | 48.8 | 49.0 |
| | | %Z | 36.6 | 51.5 | 51.0 | 51.0 | 51.2 | 51.0 |

Low and high pH speed the equilibration process. That is, the closer the pH is to neutral (7.0), the slower equilibration occurs. The pH, however, does not appear significantly to affect the equilibrium E/Z isomer ratio of roughly 1:1.

**Cited Publications**

Each of the following publication is incorporated here, in its entirety, by reference.

Atkinson, C., R. Warren, S.A. Bingham, and N.E. Day, Mammographic patterns as a predictive biomarker of breast cancer risk: effect of tamoxifen, Cancer Epidemiology, Biomarkers & Prevention, 8: 863-66 (1999).

Brisson, J., B. Brisson, G. Cote, E. Maunsell, S. Berube, and J. Robert, Tamoxifen and mammographic breast densities, Cancer Epidemiology, Biomarkers & Prevention, 9: 911-15 (2000).

Bronaugh and Maibach, Percutaneous Absorption: Drugs Cosmetics Mechanisms Methodology, Marcel Dekker 1999.

Charlier, C., A. Chariot, N. Antoine, M.P. Merville, J. Gielen, V. Castronovo, Tamoxifen and its active metabolite inhibit growth of estrogen receptor-negative MDA-MB-435 cells, 49(3): 351-8 (1995).

Fentiman, I.S., Tamoxifen and mastalgia. An emerging indication, Drugs 32: 477-80 (1986).

Fentiman, I.S., M. Caleffi, H. Hamed, and M.A. Chaudary, Dosage and duration of tamoxifen treatment for mastalgia: a controlled trial, British Journal of Surgery 75: 845-46 (1988).

Fentiman, I.S., M. Caleffi, H. Hamed, and M.A. Chaudary, Studies of tamoxifen in women with mastalgia, British Journal of Clinical Practice, Supplement 68, 43(11): 34-36 (1989)).

Gruntmanis, U. and G.D. Braunstein, Treatment of gynecomastia, Curr. Opin. Investig. Drugs, 2:643-649 (2001).

Hu, D., M.A. Hughes, G.W. Cherry, Topical tamoxifen--a potential therapeutic regimen in treating excessive dermal scarring?, Br. J. Plast. Surg., 50(6): 462-9 (1998).

Hu, D., X. Zhu, M. Xu, B. Chen, A.H. Margaret, W.C. George, The inhibitory effect of tamoxifen on human dermal fibroblast-populated collagen lattices, Zhonghua Zheng Xing Wai Ke Za Zhi, (18(3): 160-2 (2002).

Katzenellenbogen, J.A., K.E. Carlson, B.S. Katzenellenbogen, Facile geometric isomerization of phenolic non-steroidal estrogens and antiestrogens: limitations to the interpretation of experiments characterizing the activity of individual isomers, J. Steroid Biochem, 22(5): 589-96 (1985).

Kuiper, G.G.J.M., B. Carlsson, K. Grandien, E. Enmark, J. Heggblad, S. Nilsson, J. Gustafsson, Comparison of the ligand binding specificity and transcript tissue distribution of estrogen receptors $\alpha$ and $\beta$, Endocrinology, 138:863-870 (1997).

Malet C., A. Gompel, P. Spritzer, N Bricourt, NH Yaneva, I. Mowszowicz, F. Kutten and P Mauvais Jarvis, Tamoxifen and hydroxytamoxifen isomers versus estradiol effects on normal human breast cells in culture, Cancer Research, 48: 7193-7199 (1988).

Malet, C., P. Spritzer, C. Cumins, D. Guillaumin, P. Mauvais-Jarvis, F. Kuttenn, Effect of 4-hydroxytamoxifen isomers on growth and ultrastructural aspects of normal human breast epithelial (HBE) cells in culture, J. Steroid Biochem. & Mol. Bio., 82: 289-96 (2002).

Mauvais-Jarvis, P., N. Baudot, D. Castaigne, P. Banzet, and F. Kuttenn, *Trans*-4-hydroxytamoxifen concentration and metabolism after local percutaneous administration to human breast, Cancer Research, 46:1521-1525 (1986).

Nomura, Y., H. Tashiro, F. Takaeko, Effects of antiestrogens and medroxyprogesterone acetate on the clonogenic growth of tamoxifen-sensitive and resistant human breast cancer cells, Jpn. J. Cancer Chemotherapy, 12(4): 844-50 (1985).

Remington: The Science and Practice of Pharmacy, Alfonso R. Gennaro, Lippincott Williams & Wilkins, 2000, pp. 836-858.

Robertson and Katzenellenbogen, J. Org. Chem., 47: 2387 (1982).

Robertson, D.W., J.A. Katzenellenbogen, D.J. Long, E.A. Rorke and B.S. Katzenellenbogen, Tamoxifen antiestrogens. A comparison of the activity, pharmacokinetics, and metabolic activation of the *cis* and *trans* isomers of tamoxifen, J. Steroid Biochemistry, 16(1):1-13 (1982).

Son, H.J., and K.K. Oh, Significance of follow-up mammography in estimating the effect of tamoxifen in breast cancer patients who have undergone surgery, American Journal of Roentgenology, 173: 905-909 (1999).

U.S. patent No. 5,904,930

U.S. patent No. 6,172,263

U.S. patent application No. 60/433,958

U.S. patent application No. 60/433,959

U.S. patent application No. 60/458,963

**Claims**

1. A pharmaceutical composition at the equilibrium state that comprises 4-hydroxy tamoxifen, wherein about 45%-55%, about 46%-54%, about 47%-53%, about 48%-52%, about 49%-51% or about 50% of said 4-hydroxy tamoxifen exists in a Z isomeric form and the remainder of said 4-hydroxy tamoxifen exists in an E isomeric form.

2. The pharmaceutical composition of claim 1, wherein about 48%-52% of said 4-hydroxy tamoxifen exists in a Z isomeric form and the remainder of said 4-hydroxy tamoxifen exists in an E isomeric form.

3. The pharmaceutical composition of 1, wherein about 50% of said 4-hydroxy tamoxifen exists in a Z isomeric form and the remainder of said 4-hydroxy tamoxifen exists in an *E* isomeric form.

4. The pharmaceutical composition of claim 1, which is formulated for percutaneous administration.

5. The pharmaceutical composition of claim 4, which further comprises an alcohol.

6. The pharmaceutical composition of claim 4, which further comprises a penetration enhancer.

7. The pharmaceutical composition of claim 4, wherein said 4-hydroxy tamoxifen is formulated in a hydroalcoholic gel, a hydroalcoholic solution, a patch, an ointment, a cream, an emulsion (lotion), a powder or an oil.

8. The pharmaceutical composition of claim 7, wherein said 4-hydroxy tamoxifen is formulated in a hydroalcoholic gel.

9. The pharmaceutical composition of claim 8, wherein said hydroalcoholic gel comprises ethyl alcohol, isopropyl myristate, and hydroxypropylcellulose.

10. The pharmaceutical composition of claim 7, wherein said 4-hydroxy tamoxifen is formulated in a hydroalcoholic solution.

11. The pharmaceutical composition of claim 4, which is a hydroalcoholic composition further comprising a penetration enhancer, an aqueous vehicle, an alcoholic vehicle and a gelling agent.

12. The pharmaceutical composition of claim 11, which further comprises a neutralizing agent.

13. The pharmaceutical composition of claim 11, wherein the penetration enhancer comprises at least a fatty acid ester.

14. The pharmaceutical composition of claim 11, wherein said pharmaceutical composition comprises:

   a) about 0.01 % to 0.2 % by weight of 4-hydroxy tamoxifen,

   b) about 0.5 % to 2 % by weight of isopropyl myristate,

   c) about 60% to 75% by weight of absolute alcohol,

   d) about 25% to 40% by weight of aqueous vehicle,

   e) about 0.5% to 5% by weight of gelling agent,

   wherein the percentage of components are weight to weight of the composition.

15. The pharmaceutical composition of claim 14, wherein the 4-hydroxy tamoxifen constitutes about 0.01%, 0.02%, 0.03%, 0.04%, 0.05%, 0.06%, 0.07%, 0.08%, 0.09%, 0.10%, 0.11%, 0.12%, 0.13%, 0.14%, 0.15%, 0.16%, 0.17%, 0.18%, 0.19% or 0.20% by weight of the composition.

16. The pharmaceutical composition of claim 14, wherein the isopropyl myristate constitutes about 0.5%, 0.6%, 0.7%, 0.8%, 0.9%, 1.0%, 1.1%, 1.2%, 1.3%, 1.4%, 1.5%, 1.6%, 1.7%, 1.8%, 1.9% or 2.0% by weight of the composition.

17. The pharmaceutical composition of claim 14, wherein the alcohol is ethanol or isopropanol, and constitutes about 60 to 75% by weight of the composition.

18. The pharmaceutical composition of claim 14, wherein the aqueous vehicle is a phosphate buffered solution, and constitutes about 25% to 40% by weight of the composition.

19. The pharmaceutical composition of claim 14, wherein the aqueous vehicle is a phosphate buffered solution, and the pH of said pharmaceutical composition is about 4 to about 12, about 6 to about 11, about 8 to about 10, or about 9.

20. The pharmaceutical composition of claim 14, wherein the gelling agent is a polyacrylic acid, hydroxypropylcellulose or other cellulose derivative, and constitutes about 0.5% to 5% by weight of the composition.

21. The pharmaceutical composition of claim 14, which further comprises a neutralizing agent selected in the group consisting of sodium hydroxide, ammonium hydroxide, potassium hydroxide, arginine, aminomethylpropanol and tromethamine, which neutralizing agent exists at a neutralizing agent/gelling agent ratio between about 4:1 and 1:1.

22. The pharmaceutical composition of claim 14, which is packaged in a unit dose packet or in a multiple dose container with a metered pump.

**Figure 1 :** *E* **and** *Z* **isomers of Tamoxifen**

OCH$_2$CH$_2$N(CH$_3$)$_2$

C$_2$H$_5$

E tamoxifen

C$_2$H$_5$

OCH$_2$CH$_2$N(CH$_3$)$_2$

Z Tamoxifen

**Figure 2: The reversible isomerism of 4-hydroxy tamoxifen between its *Z* and *E* isomeric forms.**

Z 4-Hydroxytamoxifen          E 4-Hydroxytamoxifen

**Figure 3 : Isomer concentration ratio % for PANCHIM batch 98RD10079 at 25°C**

E/Z isomerism of PANCHIM 4-Hydroxytamoxifen batch 98RD10079 at 25°C

**Figure 4 : Isomer concentration ratio % for PANCHIM batch 98RD10079 at 30°C**

E/Z reversible isomerism of PANCHIM 4-Hydroxytamoxifen batch 98RD10079
Temperature 30°C

Legend:
- Time vs 0.02% (Z)
- Time vs 0.02% (E)
- Time vs 0.04% (Z)
- Time vs 0.04% (E)
- Time vs 0.06% (Z)
- Time vs 0.06% (E)
- Time vs 0.08% (Z)
- Time vs 0.08% (E)
- Time vs 0.01% (Z)
- Time vs 0.10% (E)

**Figure 5 : Isomer concentration ratio %  for PANCHIM  batch 98RD10079 at 40°C**

E/Z reversible isomerism of PANCHIM 4-Hydroxytamoxifen batch 98RD10079
Temperature 40°C

**Figure 6 : Isomer concentration ratio % for ICI batch Bx 17 at 40°C**

Z/E reversible isomerism of ICI 4-Hydroxytamoxifen batch Bx 17
Temperature 40°C

Legend:
- Time vs Z 0.02%
- Time vs E 0.02%
- Time vs Z 0.04%
- Time vs E0.04%
- Time vs Z 0.06 %
- Time vs E 0.06 %
- Time vs Z 0.08 %
- Time vs E 0.08 %
- Time vs Z 0.10%
- Time vs E 0.10%

**Figure 7 : Isomer concentration ratio % for solutions II to IV at 25°C**

E/Z reversible isomerism of PANCHIM batch 98RD10079 4-Hydroxytamoxifen
as a fonction of medium and temperature (25°C)

Reference solution : Gel formulation without gelification agent KLUCEL
Solution II : Reference solution without Isopropyl myristate
Solution III :Solution II with the ethyl alcohol replaced by isopropyl alcohol
Solution IV:Solution II with the ethyl alcohol/buffer concentration ratio as 50/50 instead of 66.5/33.5

**Figure 8 : Isomer concentration ratio % for solutions II to IV at 30°C**

E/Z reversible isomerism of PANCHIM batch 98RD10079 4-Hydroxytamoxifen as a fonction of medium and temperature (30°C)

Reference solution : Gel formulation without gelification agent KLUCEL
Solution II : Reference solution without Isopropyl myristate
Solution III :Solution II with the ethyl alcohol replaced by isopropyl alcohol
Solution IV: Solution II with the ethyl alcohol/buffer concentration ratio as 50/50 instead of 66.5/33.5

**Figure 9 : Isomer concentration ratio % for solutions II to IV at 40°C**

E/Z reversible isomerism of PANCHIM batch 98RD10079 4-Hydroxytamoxifen
as a fonction of medium and temperature (40°C)

Reference solution : Gel formulation without gelification agent KLUCEL
Solution 1 : Reference solution without Isopropyl myristate
Solution 2 : Solution 1 with the ethyl alcohol replaced by isopropyl alcohol
Solution 3 : Solution 1 with the ethyl alcohol/buffer concentration ratio as 50/50 instead of 72/25

**European Patent Office**

## EUROPEAN SEARCH REPORT

Application Number

EP 04 29 0762

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| E | WO 2004/054558 A (BESINS INTERNAT LAB) 1 July 2004 (2004-07-01) * the whole document * | 1-22 | A61K31/138 A61K9/08 A61K47/00 A61P35/00 |
| E | WO 2004/054557 A (LAB BESINS INTERNAT) 1 July 2004 (2004-07-01) * the whole document * | 1-22 | |
| X | US 4 919 937 A (MAUVAIS-JARVIS PIERRE ET AL) 24 April 1990 (1990-04-24) | 1-8 | |
| Y | * claim 1 * * column 3, line 29 - line 39 * | 9-22 | |
| D,X | MALET C ET AL: "EFFECT OF 4-HYDROXYTAMOXIFEN ISOMERS ON GROWTH AND ULTRASTRUCTURAL ASPECTS OF NORMAL HUMAN BREAST EPITHELIAL (HBE) CELLS IN CULTURE" JOURNAL OF STEROID BIOCHEMISTRY AND MOLECULAR BIOLOGY, ELSEVIER SCIENCE LTD., OXFORD, GB, vol. 82, no. 4/5, November 2002 (2002-11), pages 289-296, XP001181076 ISSN: 0960-0760 | 1-5 | |
| Y | * abstract * * page 293 - page 295 * | 6-22 | **TECHNICAL FIELDS SEARCHED (Int.Cl.7)** A61K |
| Y | US 6 503 894 B1 (PALATCHI OLIVIER ET AL) 7 January 2003 (2003-01-07) * claims 1-19 * * table 5 * | 6-22 | |
| Y | US 5 945 109 A (SCHMIDT ALFRED ET AL) 31 August 1999 (1999-08-31) * examples 2-4 * | 6-22 | |
| A | US 5 904 930 A (KLOKKERS KARIN ET AL) 18 May 1999 (1999-05-18) claims | 1-22 | |

The present search report has been drawn up for all claims

| Place of search | Date of completion of the search | Examiner |
|---|---|---|
| Munich | 30 August 2004 | Collura, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.** EP 04 29 0762

This annex lists the patent family members relating to the patent documents cited in the above-mentioned European search report.
The members are as contained in the European Patent Office EDP file on
The European Patent Office is in no way liable for these particulars which are merely given for the purpose of information.

30-08-2004

| Patent document cited in search report | | Publication date | Patent family member(s) | | Publication date |
|---|---|---|---|---|---|
| WO 2004054558 | A | 01-07-2004 | WO | 2004054558 A2 | 01-07-2004 |
| | | | US | 2004138314 A1 | 15-07-2004 |
| WO 2004054557 | A | 01-07-2004 | WO | 2004054557 A2 | 01-07-2004 |
| US 4919937 | A | 24-04-1990 | FR | 2558373 A1 | 26-07-1985 |
| | | | AT | 73334 T | 15-03-1992 |
| | | | DE | 3485581 D1 | 16-04-1992 |
| | | | DK | 420385 A ,B, | 17-09-1985 |
| | | | WO | 8503228 A1 | 01-08-1985 |
| | | | EP | 0151326 A1 | 14-08-1985 |
| | | | EP | 0169214 A1 | 29-01-1986 |
| | | | JP | 6067826 B | 31-08-1994 |
| | | | JP | 61500914 T | 08-05-1986 |
| US 6503894 | B1 | 07-01-2003 | AU | 8536701 A | 13-03-2002 |
| | | | AU | 8699501 A | 13-03-2002 |
| | | | AU | 9059801 A | 13-03-2002 |
| | | | BR | 0113649 A | 20-07-2004 |
| | | | CA | 2419573 A1 | 07-03-2002 |
| | | | CA | 2420895 A1 | 07-03-2002 |
| | | | CN | 1466467 T | 07-01-2004 |
| | | | CN | 1473047 T | 04-02-2004 |
| | | | EP | 1322336 A1 | 02-07-2003 |
| | | | EP | 1315502 A1 | 04-06-2003 |
| | | | EP | 1313482 A1 | 28-05-2003 |
| | | | HU | 0302921 A2 | 28-01-2004 |
| | | | JP | 2004507511 T | 11-03-2004 |
| | | | NO | 20030954 A | 28-04-2003 |
| | | | NO | 20030955 A | 28-04-2003 |
| | | | NO | 20030961 A | 25-04-2003 |
| | | | WO | 0217967 A1 | 07-03-2002 |
| | | | WO | 0217926 A1 | 07-03-2002 |
| | | | WO | 0217927 A1 | 07-03-2002 |
| | | | US | 2003050292 A1 | 13-03-2003 |
| | | | US | 2002183296 A1 | 05-12-2002 |
| | | | US | 2004002482 A1 | 01-01-2004 |
| | | | US | 2003022877 A1 | 30-01-2003 |
| | | | US | 2003232072 A1 | 18-12-2003 |
| | | | US | 2003139384 A1 | 24-07-2003 |
| US 5945109 | A | 31-08-1999 | AT | 200978 T | 15-05-2001 |
| | | | AU | 1793397 A | 22-10-1997 |
| | | | DE | 59703511 D1 | 13-06-2001 |
| | | | WO | 9736570 A1 | 09-10-1997 |
| | | | EP | 0907351 A1 | 14-04-1999 |

EPO FORM P0459

For more details about this annex : see Official Journal of the European Patent Office, No. 12/82

## ANNEX TO THE EUROPEAN SEARCH REPORT
## ON EUROPEAN PATENT APPLICATION NO.

EP 04 29 0762

This annex lists the patent family members relating to the patent documents cited in the above-mentioned European search report.
The members are as contained in the European Patent Office EDP file on
The European Patent Office is in no way liable for these particulars which are merely given for the purpose of information.

30-08-2004

| Patent document cited in search report | | Publication date | Patent family member(s) | | Publication date |
|---|---|---|---|---|---|
| US 5945109 | A | | ES 2156361 T3 | | 16-06-2001 |
| | | | JP 2001500841 T | | 23-01-2001 |
| US 5904930 | A | 18-05-1999 | DE 4407742 C1 | | 22-06-1995 |
| | | | AT 203667 T | | 15-08-2001 |
| | | | AU 694184 B2 | | 16-07-1998 |
| | | | AU 1810695 A | | 25-09-1995 |
| | | | CA 2185016 A1 | | 14-09-1995 |
| | | | DE 59509470 D1 | | 06-09-2001 |
| | | | WO 9524187 A1 | | 14-09-1995 |
| | | | EP 0748218 A1 | | 18-12-1996 |
| | | | FI 963515 A | | 06-09-1996 |
| | | | JP 9509948 T | | 07-10-1997 |
| | | | NO 963749 A | | 01-11-1996 |
| | | | PL 316177 A1 | | 23-12-1996 |
| | | | ZA 9501880 A | | 11-12-1995 |

EPO FORM P0459

For more details about this annex : see Official Journal of the European Patent Office, No. 12/82